# EUROPEAN PATENT SPECIFICATION

(11) **EP 3 397 249 B1**
(45) Date of publication and mention of the grant of the patent: **13.10.2021**
(21) Application number: 16819268.0
(22) Date of filing: 23.12.2016
(51) Int. Cl.: A61K 9/50, A61K 9/28, A61K 31/215

(54) **ENTERIC COATED ORAL PHARMACEUTICAL PREPARATION COMPRISING DIMETHYL FUMARATE**
MAGENSAFTRESISTENTES ORALES PHARMAZEUTISCHES PRÄPARAT MIT DIMETHYLFUMARAT
PRÉPARATION PHARMACEUTIQUE ORALE À ENROBAGE ENTÉRIQUE CONTENANT DU FUMARATE DE DIMÉTHYLE

(30) Priority: 31.12.2015 EP 15460145
(43) Date of publication of application: 07.11.2018
(73) Proprietor: Zaklady Farmaceutyczne Polpharma SA, 83-200 Starogard Gdanski (PL)
(72) Inventor: DROZD, Aleksandra, 55-003 Chrzastawa Mala (PL); SKOCZEN, Przemyslaw, 83-010 Straszyn (PL); CICHOCKI, Marek, 31-416 Krakow (PL)
(74) Representative: Chmurzynski, Sedzimir Lech
(86) International application number: PCT/EP2016/002176
(87) International publication number: WO 2017/114594

(56) References cited:
- WO-A1-2007/042034
- WO-A1-2015/089420
- CN-A- 104 288 774
- CN-A- 104 352 441
- US-A1- 2014 264 245

## Description

The present invention relates to an enteric coated oral pharmaceutical preparation in the form of a granulate, a pellet or a mini-tablet comprising dimethyl fumarate and having at least two coating layers, i.e. at least one inner enteric coating layer and an outer coating formed by applying a suspension comprising silicon dioxide on the at least one inner enteric coating layer. The invention relates also to the process for obtaining the enteric coated oral preparation of the present invention and to use of the preparation in the treatment of multiple sclerosis.

Multiple sclerosis is an autoimmune disease with the autoimmune activity directed against central nervous system antigens. The disease is characterized by inflammation in parts of the central nervous system, leading to the loss of the myelin, sheathing around neuronal axons (demyelination), axonal loss and the eventual death of neurons, oligodendrocytes and glial cells.

Dimethyl fumarate is used in the treatment of multiple sclerosis. A product in the form of encapsulated enteric coated mini-tablets comprising dimethyl fumarate as the sole active ingredient indicated for the treatment of relapsing remitting multiple sclerosis is marketed by Biogen Idec Ltd. under trade name Tecfidera®.

Pharmaceutical compositions comprising dimethyl fumarate are disclosed for example in EP1131065. EP1131065 discloses enteric coated pellets and mini-tablets comprising dimethyl fumarate as the sole active ingredient. A drawback of enteric coated multicompartment pharmaceutical forms is that they are prone to sticking during the initial phase of dissolution, which leads to forming of agglomerates and thus lowers dissolution. Similar behavior can be expected in vivo after administration of the preparation. Forming of agglomerates can lead to an increase of the residence time in stomach and can potentially change pharmacokinetics of the preparation.

CN104352441 and CN104288774 both disclose enteric coated pharmaceutical compositions comprising fumaric acid esters. CN104352441 relates to a preparation with an inner and outer (i.e. dual) enteric coating and CN104288774 relates to a preparation comprising a monolayer enteric coating.

The lower the sticking tendency observed in vitro the less risk that agglomeration will occur in vivo.

Surprisingly, it was found that an enteric coated pharmaceutical preparation in the form of a granulate, a pellet or a mini-tablet comprising dimethyl fumarate showing less tendency for sticking in the initial phase of dissolution can be achieved by providing the enteric coated granulate, pellet or mini-tablet with an outer coating layer formed by applying a suspension comprising silicon dioxide.

The invention relates to an enteric coated oral pharmaceutical preparation in the form of a granulate, a pellet or a mini-tablet comprising dimethyl fumarate and at least one pharmaceutically acceptable excipient present in the core of the preparation and having at least two coating layers on the core. At least one of the at least two coating layers is an inner enteric coating layer and on the at least one inner coating layer there is an outer coating formed by applying an aqueous suspension comprising silicon dioxide as the sole solid ingredient. In a preferred embodiment of the invention, the preparation has three coating layers with two inner enteric coating layers comprising different enteric film-forming polymers and an outer coating formed by applying a suspension comprising silicon dioxide.

In yet another preferred embodiment of the invention, the preparation has four coating layers with three inner enteric coating layers comprising different enteric film-forming polymers and an outer coating formed by applying a suspension comprising silicon dioxide.

In another preferred embodiment of the invention, the preparation is a granulate, preferably a granulate having a core formed by dry granulation process.

In another embodiment of the invention the preparation has three coating layers with two inner enteric coating layers comprising different enteric film-forming polymers and an outer coating formed by applying a suspension comprising silicon dioxide and the preparation is a granulate, preferably a granulate having a core formed by dry granulation process.

In another embodiment of the invention the preparation has four coating layers with three inner enteric coating layers comprising different enteric film-forming polymers and an outer coating formed by applying a suspension comprising silicon dioxide and the preparation is a granulate, preferably a granulate having a core formed by dry granulation process.

In yet another preferred embodiment of the invention, the outer coating formed by applying a suspension comprising silicon dioxide is prepared using fluid bed coating technique.

In yet another preferred embodiment of the invention, the preparation is a granulate, preferably a granulate having a core formed by dry granulation process and the outer coating formed by applying a suspension comprising silicon dioxide is prepared using fluid bed coating technique. The suspension comprising silicon dioxide is an aqueous suspension. Silicon dioxide is the sole solid ingredient forming the aqueous suspension. In yet another preferred embodiment of the invention, the coated preparation of the present invention is filled into capsules or sachets.

Another aspect of the invention relates to a process for preparation of the enteric coated oral pharmaceutical preparation in the form of a granulate, a pellet or a mini-tablet comprising dimethyl fumarate showing less tendency for sticking in the initial phase of dissolution, which process comprises the following steps:
a) providing core of the oral pharmaceutical preparation by blending dimethyl fumarate with one or more pharmaceutically acceptable excipients and
   i. granulating thus obtained blend to obtain a granulate, or
   ii. obtaining a pellet from thus obtained blend by applying extrusion and spheronization methods, or
   iii. compressing thus obtained blend into a mini-tablet;
b) coating the core of the pharmaceutical preparation with at least one enteric coating layer;
c) coating the oral preparation obtained in step b) with an aqueous suspension
comprising silicon dioxide as the sole solid ingredient. Dimethyl fumarate is preferably present in the blend obtained in step a) in the amount from 65 to 95% by weight with respect to the total weight of the blend.

The pharmaceutically acceptable excipient present in the core of the preparation is preferably selected from a group consisting of a disintegrant, an adsorbent, a lubricant and a binder and/or filler.

The disintegrant is preferably selected from croscarmellose sodium, sodium starch glycolate, crospovidone, low-substituted hydroxypropyl cellulose and mixtures thereof. The disintegrant is preferably present in the blend obtained in step a) in the amount from 4 to 12% by weight with respect to the total weight of the blend.

The adsorbent is preferably selected from silicon dioxide, aluminum magnesium silicate and mixtures thereof. The adsorbent is preferably present in the blend obtained in step a) in the amount from 0.5 to 10% by weight with respect to the total weight of the blend. The preferred adsorbent is silicon dioxide.

The lubricant is preferably selected from magnesium stearate, calcium stearate, stearic acid, sodium stearyl fumarate, glyceryl behenate, glyceryl palmitostearate and mixtures thereof. The lubricant is preferably present in the blend obtained in step a) in the amount from 0.5 to 2% by weight with respect to the total weight of the blend.

The binder and/or filler is optionally present in the blend obtained in step a) in the amount up to 20% by weight with respect to the total weight of the blend. The binder and/or filler is preferably selected from microcrystalline cellulose, mannitol, anhydrous dibasic calcium phosphate and mixtures thereof. Some of binders and fillers can be used interchangeably as they exhibit both properties.

In a preferred embodiment of the invention, the process of granulation defined in step a) point i is dry granulation. This step is preferably performed using roller compaction technique.

The process for obtaining pellets defined in step a) point ii is performed by applying extrusion and spheronization methods, however any other known methods for achieving pellets are also encompassed by the present invention.

In another preferred embodiment of the invention, the process of coating of the enteric coated preparation with a suspension comprising silicon dioxide defined in step c) is performed using fluid bed coating technique.

In yet another preferred embodiment of the invention, the process of coating with a suspension of silicon dioxide defined in step c) is performed using aqueous suspension comprising silicon dioxide. Silicon dioxide is the sole solid ingredient forming the suspension.

A further aspect of the invention relates to the enteric coated oral pharmaceutical preparation in the form of a granulate, a pellet or a mini-tablet comprising dimethyl fumarate as defined above for use in the treatment of multiple sclerosis.

The following non-limiting examples will further illustrate the invention. The skilled person would appreciate that amounts of components given in tables as a percentage [%] w/w and defining the composition of intermediate and final granulates or mixtures are equivalent to the amounts of components used for manufacturing the relevant granulates or mixtures.

### EXAMPLES

### Example 1

### Example 1.1. Preparation of enteric coated granulate comprising dimethyl fumarate

Dimethyl fumarate and croscarmellose sodium were sieved and blended. Then, sodium stearyl fumarate was sieved, added to the blend and mixed. Thus obtained blend was granulated using roller compactor. Granulate of the following composition was obtained:

| Ingredient | [%] w/w |
|---|---|
| Dimethyl fumarate | 91.60 |
| Croscarmellose sodium | 7.00 |
| Sodium stearyl fumarate | 1.40 |

The granulate was fluid bed coated using aqueous suspension of enteric film-forming polymer methacrylic acid - ethyl acrylate copolymer in the ratio of 1:1 by weight (commercially available from Evonik as Eudragit L30 D-55) and a mixture of triethyl citrate, glycerol monostearate, polysorbate 80. Enteric coated granulate of the following composition was obtained:

| Ingredient | [%] w/w |
|---|---|
| Core | |
| Dimethyl fumarate | 63.32 |
| Croscarmellose sodium | 4.85 |
| Sodium stearyl fumarate | 0.95 |

| Coating | |
|---|---|
| Methacrylic acid - ethyl acrylate copolymer (1:1) | 26.39 |
| Triethyl citrate | 2.64 |
| Glycerol monostearate | 1.31 |
| Polysorbate 80 | 0.54 |

Example 1.2. Preparation of enteric coated granulate comprising dimethyl fumarate with an outer coating formed by applying an aqueous suspension comprising silicon dioxide.

The enteric coated granulate of example 1.1 was fluid bed coated using an aqueous suspension of silicon dioxide. Enteric coated granulate with an outer silicon dioxide coating of the following composition was obtained:

| Ingredient | [%] w/w |
|---|---|
| Core | |
| Dimethyl fumarate | 62.70 |
| Croscarmellose sodium | 4.81 |
| Sodium stearyl fumarate | 0.94 |

| 1st coating layer | |
|---|---|
| Methacrylic acid - ethyl acrylate copolymer (1:1) | 26.12 |
| Triethyl citrate | 2.62 |
| Glycerol monostearate | 1.30 |
| Polysorbate 80 | 0.52 |

| 2^{nd} coating layer | |
|---|---|
| Silicon dioxide | 0.99 |

### Comparative example 1

Enteric coated granulate of example 1.1 was dry blended with silicon dioxide. The following enteric coated granulate was obtained:

| Ingredient | [%] w/w |
|---|---|
| Core | |
| Dimethyl fumarate | 62.70 |
| Croscarmellose sodium | 4.81 |
| Sodium stearyl fumarate | 0.94 |

| 1st coating layer | |
|---|---|
| Methacrylic acid - ethyl acrylate copolymer (1:1) | 26.12 |
| Triethyl citrate | 2.62 |
| Glycerol monostearate | 1.30 |
| Polysorbate 80 | 0.52 |

| Extragranular powder component | |
|---|---|
| Silicon dioxide | 0.99 |

### Comparative example 2

Enteric coated granulate of example 1.1 was dry blended with croscarmellose sodium. The following enteric coated granulate was obtained:

| Ingredient | [%] w/w |
|---|---|
| Core | |
| Dimethyl fumarate | 62.08 |
| Croscarmellose sodium | 4.76 |
| Sodium stearyl fumarate | 0.93 |

| 1^{st} coating layer | |
|---|---|
| Methacrylic acid - ethyl acrylate copolymer (1:1) | 25.87 |
| Triethyl citrate | 2.59 |
| Glycerol monostearate | 1.29 |
| Polysorbate 80 | 0.51 |

| Extragranular powder component | |
|---|---|
| Croscarmellose sodium | 1.97 |

### Comparison of dissolution

Enteric coated granulate of Example 1.1, enteric coated granulate with an outer coating comprising silicon dioxide of Example 1.2 and mixtures of enteric coated granulates with extragranular powder components of Comparative Example 1 and Comparative Example 2 were filled into hard gelatin capsules affording the dose of 240 mg per capsule and tested for dissolution in phosphate buffer (pH 6.8) after treating for 2 hours in 0.1 M solution of hydrochloric acid.

| Tested product | Dissolution in 0.1M HCI [%] | Dissolution in phosphate buffer (pH 6.8) [%] | | |
|---|---|---|---|---|
| | 120 min. | 5 min. | 10 min. | 15 min. |
| Enteric coated granulate of Ex. 1.1 | 4.4 | 12.1 | 50.2 | 77.0 |
| Enteric coated granulate with silicon dioxide coating of Ex. 1.2 | 3.2 | 15.7 | 59.6 | 80.5 |

| | | | | |
|---|---|---|---|---|
| Enteric coated granulate mixed with extragranular silicon dioxide of Comparative Ex. 1 | 4.3 | 12.4 | 51.0 | 77.8 |
| Enteric coated granulate mixed with extragranular croscarmellose sodium of Comparative Ex. 2 | 4.1 | 12.0 | 49.8 | 76.3 |

It was observed that the enteric coated granulate with silicon dioxide coating of the present invention obtained according to Example 1.2 showed much lower tendency to agglomerate in the initial phase of dissolution in the phosphate buffer and hence significantly higher dissolution when compared to the other three tested products.

### Example 2

Dimethyl fumarate, croscarmellose sodium and silicon dioxide were sieved and blended. Then, sodium stearyl fumarate was sieved, added to the blend and mixed. Thus obtained blend was granulated using roller compactor.

The granulate was fluid bed coated using a suspension of enteric film-forming polymer methacrylic acid - methyl methacrylate copolymer in the ratio of 1:1 by weight (commercially available from Evonik as Eudragit L12.5), talc and triethyl citrate in isopropanol.

The enteric coated granulate was then fluid bed coated using aqueous suspension of enteric film-forming polymer methacrylic acid - ethyl acrylate copolymer in the ratio of 1:1 by weight (commercially available from Evonik as Eudragit L30 D-55) and a mixture of triethyl citrate, glycerol monostearate, polysorbate 80.

The granulate with two layers of enteric coating was subsequently fluid bed coated using an aqueous suspension of silicon dioxide. Thus obtained enteric coated granulate with an outer silicon dioxide coating was filled into hard gelatin capsules affording the dose of 240 mg per capsule. The following composition was obtained:

| Ingredient | [%] w/w | mg/caps. |
|---|---|---|
| Core | | |
| Dimethyl fumarate | 47.52 | 240.00 |
| Croscarmellose sodium | 4.85 | 24.47 |
| Sodium stearyl fumarate | 0.51 | 2.59 |
| Silicon dioxide | 1.18 | 5.94 |

| 1^{st} coating layer | | |
|---|---|---|
| Methacrylic acid - methyl methacrylate copolymer (1:1) | 6.80 | 34.30 |
| Talc | 3.37 | 17.01 |
| Triethyl citrate | 0.65 | 3.29 |

| 2^{nd} coating layer | | |
|---|---|---|
| Methacrylic acid - ethyl acrylate copolymer (1:1) | 29.17 | 147.33 |
| Triethyl citrate | 2.93 | 14.78 |
| Glycerol monostearate | 1.46 | 7.35 |
| Polysorbate 80 | 0.57 | 2.94 |

| 3^{rd} coating layer | | |
|---|---|---|
| Silicon dioxide | 0.99 | 5.00 |

### Example 3

Dimethyl fumarate, croscarmellose sodium and silicon dioxide were sieved and blended. Then, sodium stearyl fumarate was sieved, added to the blend and mixed. Thus obtained blend was granulated using roller compactor.

The granulate was fluid bed coated using a suspension of enteric film-forming polymer methacrylic acid - ethyl acrylate copolymer in the ratio of 1:1 by weight (commercially available from Evonik as Eudragit L30 D-55) and a mixture of triethyl citrate, glycerol monostearate, polysorbate 80.

The enteric coated granulate was fluid bed coated using a suspension of enteric film-forming polymer methacrylic acid - methyl methacrylate copolymer in the ratio of 1:1 by weight (commercially available from Evonik as Eudragit L12.5), talc and triethyl citrate in isopropanol.

The granulate with two layers of enteric coating was then fluid bed coated using aqueous suspension of enteric film-forming polymer methacrylic acid - ethyl acrylate copolymer in the ratio of 1:1 by weight (commercially available from Evonik as Eudragit L30 D-55) and a mixture of triethyl citrate, glycerol monostearate, polysorbate 80.

The granulate with three layers of enteric coating was subsequently fluid bed coated using an aqueous suspension of silicon dioxide. Thus obtained enteric coated granulate with an outer silicon dioxide coating was filled into hard gelatin capsules affording the dose of 240 mg per capsule. The following composition was obtained:

| Ingredient | [%] w/w | mg/caps. |
|---|---|---|
| Core | | |
| Dimethyl fumarate | 47.52 | 240.00 |
| Croscarmellose sodium | 4.85 | 24.47 |
| Sodium stearyl fumarate | 0.51 | 2.59 |
| Silicon dioxide | 1.18 | 5.94 |

| 1st coating layer | | |
|---|---|---|
| Methacrylic acid - ethyl acrylate copolymer (1:1) | 5.84 | 29.47 |
| Triethyl citrate | 0.59 | 2.96 |
| Glycerol monostearate | 0,29 | 1.47 |
| Polysorbate 80 | 0.12 | 0.59 |

| 2^{nd} coating layer | | |
|---|---|---|
| Methacrylic acid - methyl methacrylate copolymer (1:1) | 6.79 | 34.30 |
| Talc | 3.37 | 17.01 |
| Triethyl citrate | 0.65 | 3.29 |

| 3^{rd} coating layer | | |
|---|---|---|
| Methacrylic acid - ethyl acrylate copolymer (1:1) | 23.33 | 117.86 |
| Triethyl citrate | 2.34 | 11.82 |
| Glycerol monostearate | 1.16 | 5.88 |

| | | |
|---|---|---|
| Polysorbate 80 | 0.47 | 2.35 |

| 4^{th} coating layer | | |
|---|---|---|
| Silicon dioxide | 0.99 | 5.00 |

## Claims

1. An enteric coated oral pharmaceutical preparation in the form of a granulate, a pellet or a mini-tablet comprising dimethyl fumarate and at least one pharmaceutically acceptable excipient present in the core of the preparation and having at least two coating layers on the core, **characterized in that** at least one of the at least two coating layers is an inner enteric coating layer and on the at least one inner coating layer there is an outer coating formed by applying an aqueous suspension comprising silicon dioxide as the sole solid ingredient.

2. The preparation according to claim 1, wherein the preparation has three coating layers with two inner enteric coating layers comprising different enteric film-forming polymers.

3. The preparation according to claim 1, wherein the preparation has four coating layers with three inner enteric coating layers comprising different enteric film-forming polymers.

4. The preparation according to any of the claims 1-3, wherein the preparation is a granulate, preferably a granulate having a core formed by dry granulation process.

5. The preparation according to any of the claims 1-4, wherein the outer coating formed by applying a suspension comprising silicon dioxide is prepared using fluid bed coating technique.

6. The preparation according to any of the claims 1-5, wherein the preparation is filled into capsules or sachets.

7. Process for the preparation of the enteric-coated oral pharmaceutical preparation as defined in claim 1, which comprises the following steps:
a) providing core of the oral pharmaceutical preparation by blending dimethyl fumarate with one or more pharmaceutically acceptable excipients and
i. granulating thus obtained blend to obtain a granulate, or
ii. obtaining a pellet from thus obtain blend by applying extrusion and spheronization methods, or
iii. compressing thus obtained blend into a mini-tablet;
b) coating the core of the pharmaceutical preparation with at least one enteric coating layer;
c) coating the oral preparation obtained of step b) with an aqueous suspension comprising silicon dioxide as the sole solid ingredient.

8. Process according to claim 7, wherein the granulation stage defined in step a) point i is dry granulation.

9. Process according to any of the claims 7-8, wherein coating with a suspension comprising silicon dioxide defined in step c) is performed using fluid bed coating technique.

10. An oral pharmaceutical preparation according to any of the claims 1-6 for use in the treatment of multiple sclerosis.

## Patentansprüche

1. Enterisch überzogene orale pharmazeutische Zubereitung in Form eines Granulats, eines Pellets oder einer Mini-Tablette, die Dimethylfumarat und mindestens einen pharmazeutisch unbedenklichen Hilfsstoff, die im Kern der Zubereitung vorliegen, umfasst und mindestens zwei Überzugsschichten auf dem Kern aufweist, **dadurch gekennzeichnet, dass** mindestens eine der mindestens zwei Überzugsschichten eine innere enterische Überzugsschicht ist und sich auf der mindestens einen inneren Überzugsschicht ein äußerer Überzug befindet, der durch Aufbringen einer wässrigen Suspension gebildet wird, die Siliziumdioxid als einzigen festen Bestandteil umfasst.

2. Zubereitung nach Anspruch 1, wobei die Zubereitung drei Überzugsschichten mit zwei inneren enterischen Überzugsschichten aufweist, die unterschiedliche enterische filmbildende Polymere umfassen.

3. Zubereitung nach Anspruch 1, wobei die Zubereitung vier Überzugsschichten mit drei inneren enterischen Überzugsschichten aufweist, die unterschiedliche enterische filmbildende Polymere umfassen.

4. Zubereitung nach einem der Ansprüche 1-3, wobei die Zubereitung ein Granulat ist, vorzugsweise ein Granulat mit einem durch einen Trockengranulationsverfahren gebildeten Kern.

5. Zubereitung nach einem der Ansprüche 1-4, wobei der äußere Überzug, der durch Aufbringen einer Suspension, die Siliziumdioxid umfasst, gebildet wird, unter Verwendung einer Wirbelschicht-Beschichtungstechnik zubereitet wird.

6. Zubereitung nach einem der Ansprüche 1-5, wobei die Zubereitung in Kapseln oder Sachets gefüllt wird.

7. Verfahren zur Zubereitung der enterisch überzogenen oralen pharmazeutischen Zubereitung wie in Anspruch 1 definiert, das die folgenden Schritte umfasst:
a) Bereitstellen des Kerns der oralen pharmazeutischen Zubereitung durch Mischen von Dimethylfumarat mit einem oder mehreren pharmazeutisch unbedenklichen Hilfsstoffen und
i. Granulieren der so erhaltenen Mischung, um ein Granulat zu erhalten, oder
ii. Erhalten eines Pellets aus der so erhaltenen Mischung durch Anwenden von Extrusions- und Sphäronisationsverfahren oder
iii. Verpressen der so erhaltenen Mischung zu einer Mini-Tablette;
b) Überziehen des Kerns der pharmazeutischen Zubereitung mit mindestens einer enterischen Überzugsschicht;
c) Überziehen der in Schritt b) erhaltenen oralen Zubereitung mit einer wässrigen Suspension, die Siliziumdioxid als einzigen festen Bestandteil umfasst.

8. Verfahren nach Anspruch 7, wobei die in Schritt a) Punkt i definierte Granulationsstufe eine Trockengranulation ist.

9. Verfahren nach einem der Ansprüche 7-8, wobei das in Schritt c) definierte Überziehen mit einer Suspension, die Siliziumdioxid umfasst, unter Verwendung einer Wirbelschicht-Beschichtungstechnik durchgeführt wird.

10. Orale pharmazeutische Zubereitung nach einem der Ansprüche 1-6 zur Verwendung bei der Behandlung von multipler Sklerose.

## Revendications

1. Préparation pharmaceutique orale à enrobage entérique sous la forme d'un granulé, d'une pastille ou d'un mini-comprimé comprenant du fumarate de diméthyle et au moins un excipient pharmaceutiquement acceptable présent dans le cœur de la préparation et ayant au moins deux couches d'enrobage sur le cœur, **caractérisé par le fait qu'**au moins l'une desdites au moins deux couches d'enrobage est une couche d'enrobage entérique interne et sur ladite au moins une couche d'enrobage interne il y a un enrobage externe formé par application d'une suspension aqueuse comprenant du dioxyde de silicium en tant que seul ingrédient solide.

2. Préparation selon la revendication 1, dans laquelle la préparation a trois couches d'enrobage avec deux couches d'enrobage entérique internes comprenant différents polymères filmogènes entériques.

3. Préparation selon la revendication 1, dans laquelle la préparation a quatre couches d'enrobage avec trois couches d'enrobage entérique internes comprenant différents polymères filmogènes entériques.

4. Préparation selon l'une quelconque des revendications 1 à 3, dans laquelle la préparation est un granulé, de préférence un granulé ayant un cœur formé par un procédé de granulation à sec.

5. Préparation selon l'une quelconque des revendications 1 à 4, dans laquelle l'enrobage externe formé par application d'une suspension comprenant du dioxyde de silicium est préparé à l'aide d'une technique d'enrobage en lit fluidisé.

6. Préparation selon l'une quelconque des revendications 1 à 5, dans laquelle la préparation est introduite dans des capsules ou des sachets.

7. Procédé de préparation de la préparation pharmaceutique orale à enrobage entérique telle que définie dans la revendication 1, qui comprend les étapes suivantes :
a) se procurer un cœur de la préparation pharmaceutique orale par mélange de fumarate de diméthyle avec un ou plusieurs excipients pharmaceutiquement acceptables et
i. granuler le mélange ainsi obtenu pour obtenir un granulé, ou
ii. obtenir une pastille à partir du mélange ainsi obtenu par application de procédés d'extrusion et de sphéronisation, ou
iii. comprimer le mélange ainsi obtenu en un mini-comprimé ;
b) enrober le cœur de la préparation pharmaceutique avec au moins une couche d'enrobage entérique ;
c) enrober la préparation orale obtenue à l'étape b) avec une suspension aqueuse comprenant du dioxyde de silicium en tant que seul ingrédient solide.

8. Procédé selon la revendication 7, dans lequel l'étape de granulation définie à l'étape a) point i est une granulation à sec.

9. Procédé selon l'une quelconque des revendications 7 à 8, dans lequel l'enrobage avec une suspension comprenant du dioxyde de silicium définie à l'étape c) est effectué à l'aide d'une technique d'enrobage en lit fluidisé.

10. Préparation pharmaceutique orale selon l'une quelconque des revendications 1 à 6 destinée à être utilisée dans le traitement de la sclérose en plaques.
